# EUROPEAN PATENT APPLICATION

(11) **EP 1 818 058 A2**
(43) Date of publication of application: **15.08.2007**
(21) Application number: 07102237.0
(22) Date of filing: 13.02.2007
(51) Int. Cl.: A61K 31/519, A61P 17/00

(54) **TREATMENT OF MMP-MEDIATED DERMATOLOGICAL DISEASES WITH PEMIROLAST**

(30) Priority: 13.02.2006 EP 06002867
(71) Applicant: Astion Pharma A/S, 2100 Copenhagen Ø (DK)
(72) Inventor: Weidner, Morten Sloth, DK-2830, Virum (DK)
(74) Representative: Plougmann & Vingtoft A/S

(57) **Abstract**

The invention relates to compounds for the treatment of dermatological diseases where inflammation, matrix metalloproteinases (MMPs) and peroxisome proliferator-activated receptors (PPARs) play a role in mediating the disease, such as the treatment of acne with Pemirolast or a closely related compound thereof.

## Description

### FIELD OF THE INVENTION

This invention is in the field of pharmacology and medical therapy and relates to principles and agents for the treatment of diseases where patients benefit from inhibition of matrix metalloproteinases and modulating peroxisome proliferator-activated receptors, in particular the disease acne.

### BACKGROUND OF THE INVENTION

Acne vulgaris is a skin disorder of the pilosebaceous unit of the dermis and the disorder manifests itself with non-inflammatory and inflammatory lesions. The pathogenesis of acne is multi-factorial involving at least four significant patho-physiological factors:
1) increased sebum production by the sebaceous glands, such as caused by increased activity of sebocytes via peroxisome proliferator-activated receptor ligands;
2) ductal hypercornification of the pilosebaceous unit of the dermis and hyperkeratinization (increased activity of keratinocytes);
3) inflammation; and
4) the presence of the anaerobic bacteria Propionibacterium acnes (P. acnes).

Non-inflammatory acne is often associated with the formation of a "whitehead" or a closed comedo at the skin surface. A "blackhead" may then be formed when the plug (comedo) protrudes from the opening of the duct at the skin surface.

In inflammatory acne, the area surrounding a plug (comedo) becomes inflamed and a raised, reddened papule forms on the skin. The papule may ultimately engorge with pus, forming a pustule. In severe situations, papules may penetrate extensively into surrounding tissues, forming cysts and nodules. These lesions do not heal rapidly and may result in scarring if left untreated.

There is no permanent cure for acne and particularly not for the prevention of scarring associated with acne. Treatments of more severe forms of acne usually include the use of keratolytic agents such as the retinoids and salicylic acid; antibiotics (e.g., tetracycline, benzoyl peroxide, erythromycin, clindamycin, azelaic acid) that primarily reduces the population of P. acnes; or anti-androgens such as cyproterone acetate and spironolactone that decrease sebum secretion.

It is currently considered that a number of those agents owe their beneficial effect in acne to other mechanisms than the primary effect.

For example tetracyclines, which previously were thought to reduce acne due to the inhibition of P. acnes accompanied by a reduction in sebum free fatty acids and extracellular lipases, have now been shown also to inhibit pro-inflammatory cytokines and matrix metalloproteins (Sapadin AN, Fleischmajer R. Tetracyclines: Nonantibiotic properties and their clinical implications. Journal of the American Academy of Dermatology 2006 Feb;54(2):258-65).

Furthermore, some of the retinoids (such as tretinoin), which are known to play a vital role in the treatment of acne because of their keratolytic activity, also provide anti-inflammatory activity by inhibiting the activation of toll receptors (*see* Chivot M. Retinoid therapy for acne. A comparative review. Am J Clin Dermatol 2005;6(1):13-9).

Thus, recent developments in the understanding of acne point to the fact that acne vulgaris is primarily an inflammatory disease. Therefore, future development of drugs for the treatment of acne should not only focus on reducing sebum production, P. acnes populations and hyperkeratinization, but also should aim at reducing pro-inflammatory lipids in sebum, down-regulate pro-inflammatory signals in the pilosebaceous unit, and inhibit leukotriene B4-induced accumulation of inflammatory cells. Finally, new drugs should also modulate peroxisome proliferator-activated receptors (*see* Zouboulis CC. Acne and sebaceous gland function. Clin Dermatol 2004 Sep;22(5):360-6). Inflammation seems to play a vital role in all stages of acne from the first formation of a comedone due to inflammatory cytokines over-stimulating follicular keratinization to the fierce destructive processes in inflamed acne lesions, where matrix metalloproteinases lead to tissue destruction and scar formation.

Therefore, in the search for drug agents for the treatment of acne the present inventor has recognised the strong need for therapeutic agents having multiple action points of relevance to acne and which particularly aim at inhibiting the inflammation as well as the scarring associated with acne.

It has surprisingly been found that Pemirolast (9-methyl-3-(1H-tetrazol-5-yl)-4H-pyrido[1,2-a]pyrimidin-4-one) has pharmacological properties rendering Pemirolast an ideal agent for the treatment of skin diseases, like acne, rosacea and psoriasis owing to the fact that Pemirolast has been shown to inhibit several targets, such as matrix metallo-proteinases (in particularly MMP-1), Interleukin-1α (IL-1α), Peroxisome proliferator-activated receptors (PPARα and PPAR-γ). These properties make Pemirolast a particularly advantageous drug candidate for the treatment of diseases where matrix metalloproteinases and/or Peroxisome proliferator-activated receptors together with inflammation play a role in mediating the disease.

Matrix metalloproteinases (MMPs) are a great family of endopeptidases (proteinases), which are key enzymes in normal and pathological tissue remodelling. These enzymes are synthesised and secreted by cells found in connective tissue including monocytes, macrophages and keratinocytes. The MMP family has three major subgroups, the collagenases (e.g. MMP-1), the gelatinases (e.g. MMP-2) and the stromelysins (e.g MMP-3) (Reynolds JJ, Meikle MC. The functional balance of metalloproteinases and inhibitors in tissue degradation: relevance to oral pathologies. J R Coll Surg Edinb 1997June;42(3):154-60). Acne is a disease among a plethora of diseases that have a pathogenesis associated with increased MMP expression or MMP activity in connective tissue (Papakonstantinou E, Aletras AJ, Glass E, Tsogas P, Dionyssopoulos A, Adjaye J et al. Matrix metalloproteinases of epithelial origin in facial sebum of patients with acne and their regulation by isotretinoin. J Invest Dermatol 2005 October;125(4):673-84).

Peroxisome proliferator-activated receptors (PPARs) are members of the nuclear hormone receptor superfamily of ligand-activated transcription factors that are related to retinoid, steroid and thyroid hormone receptors. PPARs play an important role in many cellular functions including lipid metabolism, cell proliferation, differentiation, adipogenesis and inflammatory signalling. PPARs have been found to interact with a number of endogenous lipids and drugs for the treatment of human metabolic diseases. There are three distinct PPAR subtypes that are the products of different genes and are commonly designated PPAR-α PPAR-δ and PPAR-γ. Each receptor shows a differential pattern of tissue expression and is activated by structurally diverse compounds.

PPARs play an important role in acne development because they regulate lipid metabolism, cell growth, and differentiation in keratinocytes and in other cells as well. PPAR-α, PPAR-δ and PPAR-γ are expressed by human keratinocytes and sebocytes, but PPAR-γ expression has been shown to be much higher in sebocytes. PPAR-γ expression has also been demonstrated in several squamous cell carcinoma cell lines. Each receptor subtype exhibits a slightly different profile of activity. PPAR-α mediates early steps of lipogenesis in both keratinocytes and sebocytes. PPAR-γ is more specific in stimulating sebocyte lipogenesis, and PPAR-δ may play a role in lipid metabolism of keratinocytes or sebocytes, depending on the circumstances (Winterfield L, Cather J, Cather J, Menter A. Changing paradigms in dermatology: nuclear hormone receptors. Clinics in Dermatology 2003;21(5):447-54).

Thus, the major part of factors involved in the pathogenesis of acne would be affected simultaneously by the treatment with Pemirolast.

So far Pemirolast has been known to be effective in suppressing or preventing various symptoms of allergic reactions such as allergic bronchial asthma and allergic rhinitis. It is an orally-active mast cell degranulation inhibitor and is today marketed for the treatment of allergic conjunctivitis. Pemirolast is known to reduce the release of histamine and the leukotrienes D4 (LTD4) and B4 (LTB4) (Kawashima T, Iwamoto I, Nakagawa N, Tomioka H, Yoshida S. Inhibitory effect of pemirolast, a novel antiallergic drug, on leukotriene C4 and granule protein release from human eosinophils. Int Arch Allergy Immunol 1994;103(4):405-9)..

Further uses of Pemirolast have been disclosed in the patent literature. For example, JP24175744A2 (KOSE CORP) describes the combined use of a vitamin D (such as cholecalciferol or ergocalciferol) and a plant extract containing an anti-allergic drug (such as pemirolast) for the treatment of skin manifestations resulting from exposure to ultraviolet-rays (sunlight).

JP23081778A2 (LION CORP) describes the combined use of a hyaluronidase activity-inhibiting component (e.g. Pemirolast) and a hyaluronic acid synthesis stimulating component, i.e. N-acetylglucosamine, N- acetylgalactosamine, D-gluconic acid, D-glucuronic acid or D-galacturonic acid for the treatment of alopecia.

JP25089345A2 (TENDOU SEIYAKU KK) describes the use of Pemirolast for the treatment of itches, swellings, inflammations and pains of various insect bites and stings.

JP4300831A2 (TOKYO TANABE CO LTD, describes the use of Pemirolast for the treatment of allergic dermatopathy. Pemirolast is formulated as a cream containing 0.01-5.00% by weight of Pemirolast or its salt, 0.2-2.0% by weight of a hydrophilic polymer, 5-20% by weight of an oily substance, 0.5-7.0% by weight of a non-ionic surfactant, 0-2% by weight of a neutralizing agent and 50-90% by weight of water.

JP24161625A2 (NOEVIR CO LTD) relates to dermatological compositions for the application to skin comprising Pemirolast and a water-soluble polyhydric alcohol, a higher fatty acid salt, a water-soluble polymer and purified water.

Thus, the use of Pemirolast for the treatment of diseases having as part of their pathogenesis abnormal sebum secretion and/or hyperkeratinisation together with inflammation is novel.

### SUMMARY OF THE INVENTION

The present inventor has shown that Pemirolast inhibits several targets, such as matrix metalloproteinases (in particularly MMP-1), inflammatory cytokines (such as TNF-α and IL-1α), PPARs (such as PPAR-α and PPAR-γ). Based on these surprising properties the inventor has recognised the potential of Pemirolast as a new drug agent for the treatment of diseases associated with enhanced sebum secretion and/or hyperkeratinisation together with inflammation, such as acne, rosacea, seborrhea, seborrheic dermatitis and psoriasis. In particularly, the multiple actions of Pemirolast renders this compound as an ideal new agent for the treatment of acne and prevention of scar formation in acne-affected skin.

Specifically, the inventor has found that with respect to acne, a beneficial effect of Pemirolast may be achieved through the simultaneous action on at least four factors:
1) Reduction of sebum secretion primarily due to inhibition of PPAR activation;
2) Reduction of hyperkeratinization due to inhibition of IL-1α:
3) Reduction of inflammation due to inhibition of inflammatory mediators, like TNF-α and IL-1α; and
4) Reduction of scar formation of inflammatory lesions due to inhibition of matrix metalloproteinases.

Accordingly, a specific first aspect of the invention relates to the use of Pemirolast or a structurally closely related compound or a pharmaceutically acceptable salt thereof for the preparation of a medicament for the treatment of acne or any clinical variant thereof.

Since the treatment of other disease may benefit from the new pharmacological profile of Pemirolast, a more general aspect of the invention relates to the use of Pemirolast or a closely related compound thereof or a pharmaceutically acceptable salt thereof as a medicament for the treatment of a disease characterised by the presence of inflammation together with activity of MMPs and/or PPAR ligands from the group consisting of acne, acne scarring, psoriasis, chronic ulcers, scars, burns, skin cancer, skin ageing, rosacea, seborrhea and seborrheic dermatitis.

In an alternative first aspect, the invention relates to methods for the treatment of acne or any clinical variant thereof or acne scarring or any other disease as mentioned in the preceding paragraph; psoriasis, chronic ulcers, scars, burns, skin cancer, skin ageing, rosacea, seborrhea and seborrheic dermatitis , comprising administering to a mammal an effective amount of Pemirolast or a structurally closely related compound thereof including a pharmaceutically acceptable salt thereof. The administration is preferably effected by cutaneous administration to the skin.

A second aspect of the invention features a topically administrable pharmaceutical composition formulated for the application to skin comprising as the therapeutically active ingredient Pemirolast or a closely related compound thereof or a pharmaceutically acceptable salt thereof in a therapeutically effective amount of at least 0.1% and further comprising a dermatological acceptable carrier or vehicle.

### DETAILED DESCRIPTION OF THE INVENTION

As used herein, the following definitions are applied:

The term "skin" is meant to include skin of the entire body including the scalp, the forehead, the head, arms, legs, breast and so forth. The term "skin" is also meant to include various layers of the skin, such as stratum corneum, epidermis and dermis.

The term "mucosa" is, in the context of the present invention, meant to include mucosa of the buccal cavity.

The term "cutaneous administration" is in the context of the present invention meant to include topical administration to the skin, such as topical administration to the skin for the local treatment of a disease of the skin.

The term "buccal cavity" is meant to define a mucosa membrane of the buccal cavity, such as the gingival tissues.

The phrase "a subject in need thereof" refers to a person having the risk or suffering from a disease mentioned herein.

The term "subject" for purposes of treatment includes any subject, but is preferably a subject who is in need of the treatment of a MMP mediated dermatological disease mentioned herein, in particularly acne. For purposes of prevention, the subject is any subject, and preferably is a subject that is at risk for, or is predisposed to, developing such a dermatological disease. The subject is typically an animal, and yet more typically is a mammal. "Mammal", as used herein, refers to any animal classified as a mammal, including humans, domestic and farm animals, zoo, sports, or pet animals, such as dogs, horses, cats, cattle, etc. Preferably, the mammal is a human.

The terms "treat", "treating" and "treatment", as used herein, are meant to include alleviating or abrogating a disease as defined herein or its attendant symptoms and alleviating or eradicating the cause of the disease itself.

The terms "prevent", "preventing" and 'prevention", as used herein, refer to a method of delaying or precluding the onset of symptoms of a disease as defined herein, such as preventing the reoccurrence of acne-affected skin.

The phrase "therapeutically effective amount" refers to the amount of Pemirolast or a closely related compound as defined herein that will elicit the biological or the medical response of a tissue, system, animal or human that is being sought by the researcher, veterinarian, medical doctor or other clinician. The phrase "therapeutically effective amount" includes that anamount of said compounds that, when administered, is sufficient to prevent development of, or alleviate to some extent, one or more of the symptoms of the condition or disorder being treated. The therapeutically effective amount will vary depending on the compound, the disease and its severity and the age, weight, etc., of the mammal to be treated.

The phrase "Pemirolast or a closely related compound thereof or a pharmaceutically acceptable salt thereof" is defined in the section with the heading "Active ingredient of the invention". Where the application text only mentions Pemirolast, it is the intention also to state "or a closely related compound thereof or a pharmaceutically acceptable salt thereof", but for ease reading there is applied the short term Pemirolast.

### Uses and Methods

### Acne and acne scarring

As mentioned above, Pemirolast is a drug agent with a totally revised pharmacological profile because Pemirolast has changed from being a mast cell stabilisator to a drug agent capable of inhibiting several new classes of targets of significant pharmacological importance:
- MMPs, such as MMP-1;
- PPARs, such as PPARα and PPAR-γ; and
- Inflammatory cytokines, such as IL-1α and TNF-α.

Since all these targets are of strong relevance to the pathogenesis of acne, the invention first of all relates to the use of Pemirolast or a structurally closely related compound or a pharmaceutically acceptable salt thereof for the preparation of a medicament for the treatment of acne or any clinical variant thereof or acne scarring. This also means that the invention encompasses methods for the treatment of acne or any clinical variant thereof or acne scarring, comprising administering to a mammal an effective amount of Pemirolast or a structurally closely related compound including a pharmaceutically acceptable salt thereof.

The phrase "any clinical variant thereof" is meant to encompass acne vulgaris, inflammatory acne, non-inflammatory acne, acne fulminans, nodular papulopustular acne, and acne conglobata.

In one embodiment, the treatment is directed to the prevention or treatment of acne vulgaris.

As mentioned above, acne is often accompanied by scarring (scar formation) and one embodiment of the invention relates to the regulation, prevention or treatment of acne scarring.

Acne can be graded according to the severity of symptoms. Thus, the term "acne includes mild acne, moderate acne, severe acne and very severe acne. Mild acne refers to the presence of few skin lesions, such as few to several papules/pustules and nonodules; moderate acne refers to the presence of several to many papules/pustules, few to several nodules and numerous and extensive comedones; severe acne refers to numerous and/or extensive papules/pustules, many persistent or recurrent nodules large and very extensive comedones, ongoing scarring, persistent purulent and/or serosanguinous drainage from lesions, presence of sinus tracts; and very severe acne includes conditions termed acne conglobata, acne fulminans and acne inversa (follicular occlusion triad).

Thus, it should be understood that in an interesting embodiment of the invention the treatment is useful to reduce symptoms, such as comedones, papules, pustules, inflamed nodules, superficial pus-filled cysts, skin lesions and dermal inflammation of the skin of a subject suffering from such symptoms, such as a subject suffering from acne.

More specifically, one embodiment of the invention relates to the treatment of mild acne, moderate acne, severe acne and very severe acne including the prevention of acne scarring.

### Other diseases mediated by inflammation together with MMP activity and/or PPAR binding

It should be understood that the novel pharmacological profile of Pemirolast may justify the use of Pemirolast in the treatment of other diseases where MMP activity and/or PPAR ligands are known to play a role in addition to the inflammation in mediating the disease.

Therefore, in general the invention relates to the use of Pemirolast or a closely related compound thereof or a pharmaceutically acceptable salt thereof as a medicament for the treatment of a disease characterised by the presence of inflammation together with activity of MMPs and/or PPAR ligands from the group consisting of acne, acne scarring, psoriasis, chronic ulcers, scars, burns, skin cancer, skin ageing, rosacea, seborrhea and seborrheic dermatitis.

MMPs are known to play a role in many topical disorders in which extracellular protein degradation/destruction occurs. For a general review see (Kahari VM, Saarialho-Kere U. Matrix metalloproteinases in skin. Exp Dermatol 1997 October;6(5):199-213). To be mentioned here is pathological conditions associated with increased MMP expression or MMP activity in cells found in connective tissue, such as chronic ulcers (Miyoshi H, Kanekura T, Aoki T, Kanzaki T. Beneficial effects of tissue inhibitor of metalloproteinases-2 (TIMP-2) on chronic dermatitis. J Dermatol 2005 May;32(5):346-53*),* psoriasis *(*Flisiak I, Mysliwiec H, Chodynicka B. Effect of psoriasis treatment on plasma concentrations of metalloproteinase-1 and tissue inhibitor of metalloproteinase-1. J Eur Acad Dermatol Venereol 2005 July;19(4):418-21), oral pathologies, such as gingivitis and periondontitis (J R Coll Surg Edinb 1997 June;42(3):154-60), skin cancer (Ntayi C, Hornebeck W, Bernard P. [Involvement of matrix metalloproteinases (MMPs) in cutaneous melanoma progression]. Pathol Biol (Paris) 2004 April;52(3):154-9*),* such as non-melanoma skin cancers, basal (BCC) and squamous (SCC) cell carcinoma (Kerkela E, Saarialho-Kere U. Matrix metalloproteinases in tumor progression: focus on basal and squamous cell skin cancer. Exp Dermatol 2003 April;12(2):109-25), tumor invasion and metastasis (Sato H, Takino T, Miyamori H. Roles of membrane-type matrix metalloproteinase-1 in tumor invasion and metastasis. Cancer Sci 2005 April;96(4):212-7).

Furthermore, MMPs play an important role in various physiological situations where the extracellular matrix is degraded or rebuilt (i.e. proteolytic remodeling of extracellular matrix), including developmental tissue morphogenesis, tissue repair, and angiogenesis (Kahari VM, Saarialho-Kere U. Matrix metalloproteinases in skin. Exp Dermatol 1997 October;6(5):199-213). Particularly, MMPs play a role in connective tissue remodeling (Abraham D, Ponticos M, Nagase H. Connective tissue remodeling: cross-talk between endothelins and matrix metalloproteinases. Curr Vasc Pharmacol 2005 October;3(4):369-79), for example in connection with an alteration of the collagen fibres as a result of degradation of collagen tissue by MMPs leading to the skin having a soft and wrinkled appearance. MMPs also play a role in skin ageing, such as ageing of sun-exposed skin (Amano S, Ogura Y, Akutsu N, Matsunaga Y, Kadoya K, Adachi E et al. Protective effect of matrix metalloproteinase inhibitors against epidermal basement membrane damage: skin equivalents partially mimic photoageing process. Br J Dermatol 2005 December;153 Suppl 2:37-46) and skin ageing in smokers (Lahmann C, Bergemann J, Harrison G, Young AR. Matrix metalloproteinase-1 and skin ageing in smokers. Lancet 2001 March 24;357(9260):935-6*)* and UV-induced skin ageing (Rittie L, Fisher GJ. UV-light-induced signal cascades and skin aging. Ageing Res Rev 2002 September;1(4):705-20).

Thus, Pemirolast may also be used as a medicament in the treatment of psoriasis, chronic ulcers, scars, burns, skin cancer such as non-melanoma skin cancers basal (BCC) and squamous (SCC) cell carcinoma, skin ageing, such as skin ageing caused by smoking, sun or UV light or cutaneous wrinkling.

As mentioned, one embodiment of the invention relates to the treatment of psoriasis. There is reason to expect that Pemirolast may be an ideal candidate also for treating psoriasis. Recent knowledge points to the fact that treatment of psoriasis should target keratinocyte proliferation through the effect on PPARs, target angiogenesis through action on matrix metalloproteases and target inflammation by inhibition of TNF-α and IL-1α (Hamminga EA, van der Lely AJ, Neumann HAM, Thio HB. Chronic inflammation in psoriasis and obesity: Implications for therapy. Medical Hypotheses 2006;67(4):768-73 and Bayliffe AI, Brigandi RA, Wilkins HJ, Levick MP. Emerging therapeutic targets in psoriasis. Current Opinion in Pharmacology 2004 Jun;4(3):306-10 *and* Mee JB, Cork MJ, di Giovine FS, Duff GW, Groves RW. Interleukin-1: A key inflammatory mediator in psoriasis? Cytokine 2006 Jan 21;33(2):72-8). Further evidence for the view that Pemirolast will work in the treatment of psoriasis emerges from the fact that retinoids are used in the treatment of psoriasis and retinoids exhibit the same effect on keratinocyte proliferation that results from PPARs.

Furthermore, there is evidence supporting the view that Pemirolast may be used in the treatment of skin ageing because both MMP inhibitors are suggested as good candidates in treatment of skin ageing.

The term "Ageing skin" refers to skin that naturally begins to lose its firmness or to skin where external factors such as UV light and sun exposure promote the ageing of the skin. Medically, such processes are called "actinic elastosis" or "photoaging". As skin ages it takes on a thin and transparent appearance. Sometimes freckles or age spots (also called liver spots) begin to develop, as well as spider veins, wrinkles and fine lines around the eyes and mouth. Skin that was once supple and hydrated can begin to feel dry, itchy and appear to sag as we age because of damage to the fibers underneath the skin's surface.

Skin ageing may be caused by smoking, such as in skin ageing in smokers, or it may be caused by UV-induced damage to skin connective tissue, such as in photoageing. Aged skin may also be caused by intrinsic ageing that refer to an age-dependent deterioration of skin functions, also termed chronologically aged skin.

In another embodiment, Pemirolast may be used in the treatment of Rosacea. Rosacea is a disease which to some extent resembles acne, and tetracyclines are used in the treatment of both diseases (The current status of subantimicrobial dose doxycycline therapy in the management of rosacea and acne. Journal of the American Academy of Dermatology 2005 Mar;52(3, Supplement 1):23). It has been discovered that tetracyclines apart from their antibacterial effect also inhibit MMPs (MMP-1).

Rosacea is characterised by angiogenesis and inflammation. Angiogenesis relates to the process of formation of new blood vessels and include benign conditions (e.g. rosacea) and malignant processes (e.g. cancer). Matrix-degrading enzymes, present in the extracellular matrix of tissues, facilitate angiogenesis by allowing new blood vessels to penetrate into the matrix. MMPs represent one such class of enzymes involved in this process (Sapadin AN, Fleischmajer R. Tetracyclines: Nonantibiotic properties and their clinical implications. Journal of the American Academy of Dermatology 2006 Feb;54(2):258-65).

Thus, rosacea is another disease that may be treated well with Pemirolast.

Like Rosacea, angiogenesis and inflammation is characteristic for a number of other diseases (Sapadin AN, Fleischmajer R. Tetracyclines: Nonantibiotic properties and their clinical implications. Journal of the American Academy of Dermatology 2006 Feb;54(2):258-65). Therefore, according to this invention various embodiments encompass the use of Pemirolast in the treatment of bullous dermatoses, pyoderma gangrenosum, sarcoidosis, aortic aneurysms, cancer metastasis, periodontitis, and autoimmune disorders such as rheumatoid arthritis and scleroderma.

Specifically, some embodiments of the invention relate to the use of Pemirolast as a medicament in the treatment of oral pathologies, such as gingivitis and periondontitis.

Another condition where MMP activity plays a role is with the formation of scars in collagen-containing tissue. Therefore, one embodiment of the present invention relates to the regulation, prevention or treatment of scar formation in skin. The reduction of scarring of the skin may be a desire with respect to both pathological conditions, such as scarring in fibrotic disorders, or with respect to cosmetic considerations, such as acne scarring.

The phrase "scar formation in the skin" or "scarring of the skin" refers to the formation of an abnormal collagen-containing morphological structure due to a previous injury or wound in the collagen-containing tissue of the skin. Most scars consist of abnormally organised collagen fibers and also excess of collagen. There are lots of different causes of scarring - accidents, surgery, skin diseases, burns, acne, infection and traumas in general - but not all scars are the same. Below are some of the different types of scars that occur:
■ Flat, Pale Scars - formed as a result of the body's natural healing process.
■ Sunken Scars - formed due to the skin being attached to deeper structures (such as muscles) or to loss of underlying fat. Sunken scars are depressed below the skin surface and they are usually the result of an injury.
■ Hypertrophic Scars - formed when the body is overproducing collagen in connection with wound healing. Hypertrophic scars are elevated above the normal surface of the skin and contain excessive collagen arranged in an abnormal pattern.
■ Keloid Scars - formed as the result of an imbalance in the production of collagen in a healing wound. Keloid scars are not only elevated above the surface of the skin, but also extends beyond the boundaries of the original injury and they can continue to grow indefinitely. In a keloid scar there is excessive connective tissue which is organised in an abnormal fashion predominately in whirls of collagenous tissue. Keloid scars are particularly common in Africo-Carribean and Mongoloid races. Keloid scars can result from any type of injury to the skin, including scratches, injections, insect bites and tattoos.
■ Acne Scars are formed in acne-affected skin. The scar may be a sunken scarring or become keloid. Similar scars may also be seen in persons infected with chicken pox.
■ Stretched Scars - occurring when the skin around a healing wound is put under tension during the healing process. This type of scarring may follow injury or surgery. Initially, the scar may appear normal but can widen and thin over a period of weeks or months. This can occur where the skin is close to a joint and is stretched during movement or may be due to poor healing due to general ill health or malnutrition.
■ Stretch Marks - formed when the skin is stretched rapidly, for example during pregnancy or the adolescent growth spurt.

Thus, in various embodiments of the invention the treatment of scars include the regulation, prevention or treatment of hypertrophic scars, keloid scars, acne scars, stretched scars or stretch marks in skin.

The application of Pemirolast in diseases where PPAR rather than MMP activity is thought to play a role in mediating the disease or condition is Seborrhea, which is characterised by superfluous sebum production just like acne. Therefore, in another embodiment of the invention, Pemirolast is used as a medicament in the treatment of Seborrhea.

In still another embodiment of the invention, Pemirolast is for use as a medicament in the treatment of Seborrheic dermatitis, which is a disease characterised by hyperkeratinisation and release of inflammatory cytokines.

A still another condition where Pemirolast might be used is in the treatment of skin cancer in that both MMP activity and PPAR-γ expression has been demonstrated in several squamous cell carcinoma cell lines. Degradation and remodeling of the extracellular matrix is a crucial event in tumor invasion and various MMPs have been implicated in the tumour progression of cancer cells in human tissue, such as in basal cell carcinoma. In particularly squamous cell carcinomas of skin and buccal cavity are characterised by high ability to invade locally by a MMP mediated process. The degradation of epithelial basis membrane is a prerequisite for the invasion of epithelial melanocytic tumor cells into the dermis. Melanoma is a highly invasive skin cancer form, where the degradation of matrix proteins in the dermis is a key step of melanoma invasion.
Thus, in another embodiment of the invention, Pemirolast is for use as a medicament in the treatment of prevention or treatment of cancers affecting the skin, epidermis, dermis or a mucosa, such as basal cell carcinoma, squamous cell carcinoma and melanoma. More particularly, methods and uses of the invention relate to preventing, progression or tumour cell invasion of tumours affecting the skin, epidermis, dermis or a mucosa, such as basal cell carcinomas, squamous cell carcinomas and melanomas.

Whilst the above considerations mainly apply to conditions, disorders or diseases of man it will be appreciated that wound healing, scarring and fibrotic disorders can also be problematic in other animals, particularly veterinary or domestic animals (e.g. horses, cattle, dogs, cats etc). For instance abdominal wounds or adhesions are a major reason for having to put down horses (particularly race horses), as tendon and ligament damage leads to scarring or fibrosis.

### Active ingredient of the invention

This invention relates to new therapeutic applications and dermatological compositions of Pemirolast (9-methyl-3-(1H-tetrazol-5-yl)-4H-pyrido[1,2-a]pyrimidin-4-one) and structurally closely related compounds to Pemirolast as well as the pharmaceutically acceptable salts thereof. Such closely related compounds are in general 3-Tetrazolopyrido[1,2-a]pyrimidin-4-one derivatives, as described in US 4,122,274, and where one or more of the hydrogen atoms at positions 6, 7, 8 or 9 are substituted.

### Pemirolast has the following structure I:

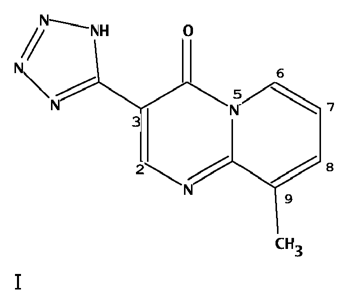

The phrase "structurally related compounds to Pemirolast" or "Pemirolast or a closely related compound thereof" are interchangeable phrases, which are meant to refer to 3-Tetrazolo-7,9-substituted-pyrido[1,2-a]pyrimidin-4-one derivatives" that have a close structural similarity to Pemirolast and which are further defined below.

In the context of the present invention, such closely related compounds are thought to encompass 3-Tetrazolo-pyrido[1,2-a]pyrimidin-4-one derivatives, where none, one or two hydrogen atoms are substituted in the 6, 7, 8 or 9 position.

A 3-Tetrazolo-pyrido[1,2-a]pyrimidin-4-one derivative of the present invention is defined by formula II 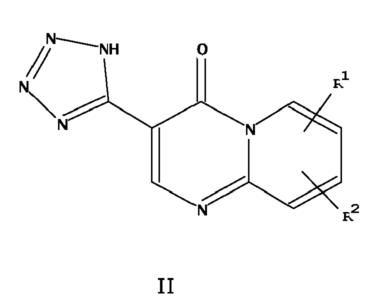 wherein R¹ and R², which may be the same or different, each independently designate radicals selected from the group consiting of hydrido (H), optionally substituted C₁₋₆-alkyl, C₄₋₆-cycloalkyl, C₂₋₆-alkenyl, C₂₋₆-alkynyl, C₁₋₆-alkoxyl, phenyl, C₇₋₁₄alkaryl, C₇₋₁₄ alkheterocyclyl, carboxyl (COOH), carboxyl derivative (COOR'), cyano (CN), CF₃, halogen (Br, Cl, Fl, I), hydroxy (OH), hydroxy derivative (OR'), primary amino (NH₂), secondary amino (NHR'), tertiary amino (NR'R"), carboxy (CO), carboxy derivative (CO- R'), and wherein R' and R" independently defines a radical selected from C₁₋₆-alkyl, C₄₋₆-cycloalkyl, C₂₋₆-alkenyl, C₂₋₆-alkynyl, C₁₋₆-alkoxyl, phenyl, C₇₋₁₄ alkaryl, C₇₋₁₄ alkheterocyclyl.

Within the above mentioned group of compounds, a preferred subgroup comprises those compounds, wherein R¹ and R², which may be the same or different, each independently designate radicals selected from hydrido (H), optionally substituted C₁₋₆-alkyl, C₄₋₆-cycloalkyl, C₂₋₆-alkenyl, C₁₋₆-alkoxyl, phenyl, benzyl, cyano (CN), CF₃, halogen (Br, Cl, Fl, I), hydroxy (OH), primary amino (NH₂), secondary amino (NHR'), tertiary amino (NR'R"), and wherein R' and R" independently defines a radical selected from C₁₋₆-alkyl, C₄₋₆-cycloalkyl and C₂₋₆-alkenyl.

The R¹ and R² substituents of compounds of structure II may be located at any of the positions 6, 7, 8 or 9 of the pyrido[1,2-a]pyrimidine ring system.
However, preferably, the R¹ and R² substituents are located at positions 7 and/or 9, resulting in derivatives of structure III, wherein R¹ and R² are as defined above:

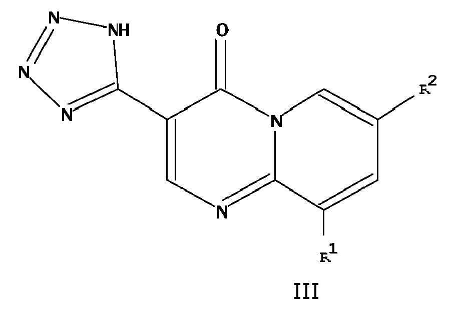

In a currently interesting embodiment of the invention, the 3-(1H-tetrazol-5-yl)-4H-pyrido[1,2-a]pyrimidin-4-one derivative is defined by the above mentioned formula III, wherein R¹ and R², which may be the same or different, each independently designate radicals selected from hydrido (H), optionally substituted C₁₋₆-alkyl, C₄₋₆-cycloalkyl, C₂₋₆-alkenyl, C₁₋₆-alkoxyl, phenyl, benzyl, cyano (CN), CF₃, halogen (Br, Cl, FI, I), hydroxy (OH), primary amino (NH₂), secondary amino (NHR'), tertiary amino (NR'R"), and wherein R' and R" independently defines a radical selected from C₁₋₆-alkyl, C₄₋₆-cycloalkyl and C₂₋₆-alkenyl.

While the R¹ and R² substituents in the compounds of formula II or III may be located at any of positions 6, 7, 8 or 9 of the pyrido[1,2-a]pyrimidine ring system, the 7- and 9-substituted compounds are preferred. However, the most preferred embodiments are monosubstituted derivatives of 3-(1H-tetrazol-5-yl)-4H-pyrido[1,2-a]pyrimidin-4-one derivatives of structures II or III, most preferably compounds of the above mentioned formulas II or III having the substituent R¹ or R² at the 9-position. That is to say that the most preferred embodiments, encompass those, wherein either R¹ or R² is hydrido (H).

C₁₋₆-alkyl is meant to define saturated, straight chained or branched alkyl radical containing from 1 to 6 carbon atoms, e.g. all alkyl radicals from methyl up to hexyl including all isomers thereof, e.g. iso-butenyl.

C₂₋₆-alkenyl defines unsaturated straight chained or branched alkylene radicals containing from 2 to 6 carbon atoms, e.g. 1- or 2-propenyl, 1-, 2- or 3-butenyl and the like and isomers thereof.

C₂₋₆-alkynyl defines unsaturated chained or branched alkynyl radicals containing from 2 to 6 carbon atoms, e.g. ethynyl, 1- or 1-propynyl, 1-, 2- or 3-butynyl and the like and isomers thereof.

C₁₋₆-alkoxyl means alkoxy radicals containing up to 6 and preferably up to 4 carbon atoms, e.g. methoxy, ethoxy, propoxy etc.

C₄₋₇-cycloalkyl means a cycloalkane having from 4 to 7 carbon atoms, such as cyclobutane, cyclopentane and cyclohexane.

The term " C₇₋₁₄ alkaryl " embraces aryl-substituted alkyl radicals such as benzyl, diphenylmethyl, phenethyl, and diphenethyl having from 7 to 14 carbon atoms.
The term "C₇₋₁₄ alkheterocyclyl" designates an alkyl substituted heterocyclic group having from 7 to 14 carbon atoms in addition to one or more heteroatoms, N, S, P, or O, (e.g., 3-furanylmethyl, 2-furanylmethyl, 3- tetrahydrofuranylmethyl, or 2-tetrahydro-furanylmethyl. Nonlimiting examples of heterocylics are pyrrolidinyl, tetrahydrofuryl, tetrahydrofuranyl, pyranyl, purinyl, tetrahydropyranyl, piperazinyl, piperidinyl, morpholino, thiomorpholino, tetrahydropyranyl, imidazolyl, pyrolinyl, pyrazolinyl, indolinyl, dioxolanyl, or 1,4-dioxanyl, aziridinyl, furyl, furanyl, pyridyl, pyridinyl, pyridazinyl and pyrimidinyl.

The groups, C₁₋₆-alkyl, C₄₋₆-cycloalkyl, C₂₋₆-alkenyl, C₂₋₆-alkynyl, C₁₋₆-alkoxyl, C₇₋₁₄ alkaryl, and C₇₋₁₄ alkheterocyclyl may optionally be mono or di-substituted with primary amino (NH₂), secondary amino (NHR'), tertiary amino (NR'R"), OH, cyano, nitro and halogen, wherein R' and R" are as defined above.

The term halogen defines bromine, chlorine, fluorine and iodine. The term "hydrido" designates a single hydrogen atom (H).

The groups, C₁₋₆-alkyl, C₁₋₉-alkyl, C₂₋₆-alkenyl, C₂₋₉-alkenyl, C₂₋₆-alkynyl and C₂₋₉-alkynyl may optionally be mono-substituted with CN, CO, CHO, COR⁵, halogen, OH, OR' NH₂, NHR', NR'R" and nitro, wherein R⁵, R⁶, R' and R" are as defined above.

The term halogen defines bromine, chlorine, fluorine and iodine. The term "hydrido" designates a single hydrogen atom (H).

Compounds of structures I, II and II encompass "pharmaceutically acceptable salts thereof". Since the compounds of this invention are amphoteric in nature, they can be converted to salts of either acids or bases by treating said compounds with a substantially equimolar amount of a chosen acid or base in an aqueous solution or in a suitable organic solvent such as methanol or ethanol. In the present context, the phrase "a pharmaceutically acceptable salt" encompasses a base addition salt derived from the reaction of a free acidic hydrogen atom of structures I, II and III with inorganic bases (hydroxides) or organic bases or/and an acid addition salt derived from the reaction of a basic nitrogen of structures I, II and II with pharmaceutically acceptable acids. Thus, it might be understood that Pemirolast or derivatives of structures II or III may be provided in the form of an acid addition salt or a base addition salt or in the form of a double salt of mixed acid addition and base addition salt.

Examples of pharmaceutically acceptable salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic or phosphorous acids and the like, as well as the salts derived from relatively non-toxic organic acids like acetic, propionic, isobutyric, maleic, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galacturonic acids and the like.

Where Pemirolast or a derivative according to structures II or III is provided in the form of a salt it is meant to include a pharmaceutically acceptable solvate, which may be a hydrate (comprising from half a mole of H₂O up to about 10 moles of H₂O per mole of salt) or comprise other solvents of crystallization such as alcohols.

Examples on base addition salts encompass Na, K, Ca, Mg, Cu, Zn and Mn salts. Typically organic bases for use in the preparation of a base addition salt are primary, secondary or tertiary amines including alkylphenylamine, ammonia, 2-aminoethanol, aminopyrimidine, aminopyridine, arginine, benethamine, benzathine, betaine, caffeine, choline, deanol, diethanolamine, diethylamine, 2-diethylaminoethanol, 2-dimethylaminoethanol, ethylenediamine, N- ethylmorpholine, N-ethylpiperidine, glucamine, glucosamine, glycinol, hydrabamine, imidazol, isopropylamine, meglumine, methylglucamine, morpholine, piperazine, piperidine, procaine, purine, pyrrolidine, theobromine, thiamine, triethanolamine, triethylamine, trimethylamine, tripropylamine, tromethamine, spermidine, and the like). Furthermore, base addition salts may be derived from the reaction with natural amino acids such as with glycine, alanine, valine, leucine, isoleucine, norleucine, tyrosine, cystine, cysteine, methionine, proline, hydroxy proline, histidine, omithine, lysine, arginine, serine, threonine, and phenylalanine and with unnatural amino acids such as D-isomers or substituted amino acids; guanidine, substituted guanidine wherein the substituents are selected from nitro, amino, alkyl, alkenyl, alkynyl, ammonium or substituted ammonium salts and aluminum salts.

In a preferred embodiment of the invention, the pharmaceutically acceptable salt is a salt between zinc and Pemirolast or a closely related compound thereof.

As the zinc compound may employed any member selected from the group consisting of water soluble, poorly water soluble and water insoluble zinc salts, compounds and complexes, such as zinc acetate, zinc bacitracin, zinc bromide, zinc cysteate, zinc caprylate, zinc chloride, zinc citrate, zinc fluoride, zinc formate, zinc glycinate, zinc iodate, zinc lactate, zinc nitrate, zinc nitrite, zinc oleate, zinc oxalate, zinc oxide, zinc permanganate, zinc peroxide, zinc phenolsulfonate, zinc phosphate, zinc propionate, zinc pyrophosphate, zinc ricinoleate, zinc salicylate, zinc selenate, zinc silicate, zinc selenide, zinc sulfate, zinc stearate, zinc sulfide, zinc tannate, zinc tartrate, zinc valerate, zinc peptides, and zinc protein complexes.

Those skilled in the art will appreciate that the compounds represented by formulas I, II and III contain a tautomeric hydrogen atom and the compounds are thus capable of existing in the 1H-tetrazol-5-yl form and the 2H-tetrazol-5-yl form. This invention embraces both forms, but for the sake of convenience, structures I, II and III have been arbitrarily selected to describe the present compounds.

The compounds of the present invention may be prepared by the methods set forth in the patent US4122274.

Current interesting derivatives are:
7-ethyl-3-(1H-tetrazol-5-yl)-4H-pyrido[1,2-a]pyrimidin-4-one, or a pharmaceutically acceptable salt thereof.
   7-n-butyl-3-(1H-tetrazol-5-yl)-4H-pyrido[1,2-a]pyrimidin-4-one, or a pharmaceutically acceptable salt thereof.
   7-phenyl-3-(1H-tetrazol-5-yl)-4H-pyrido[1,2-a]pyrimidin-4-one, or a pharmaceutically acceptable salt thereof.
   7-chloro-3-(1H-tetrazol-5-yl)-4H-pyrido[1,2-a]pyrimidin-4-one, or a pharmaceutically acceptable salt thereof.
   9-methyl-3-(1H-tetrazol-5-yl)-4H-pyrido[1,2-a]pyrimidin-4-one, or a pharmaceutically acceptable salt thereof (Pemirolast).
   3-(1H-tetrazol-5-yl)-4H-pyrido[1,2-a]pyrimidin-4-one, or a pharmaceutically acceptable salt thereof.
   9-ethyl-3-(1H-tetrazol-5-yl)-4H-pyrido[1,2-a]pyrimidin-4-one, or a pharmaceutically acceptable salt thereof.

### Manner of administration and doses

An active ingredient of the invention (Pemirolast or a closely related compound thereof or a pharmaceutically acceptable salt thereof) may be administered to a subject through any route of administration resulting in either local presence of the active ingredient in skin or in buccal mucous or systemic presence of the active ingredient.

The phrase "local presence of the active ingredient in skin or mucous" is meant to include topical administration of the active ingredient to skin or mucosa, such as mucosa of the eye, buccal cavity, nasal cavity, or intestinal tract with the presumption that systemic uptake of the active ingredient is limited or nil. Thus, it is intended that less than 15% by weight, such as less than 10%, 8%, 5% and 3% by weight, of the topically administered active ingredient of the invention may either enter the blood stream or be recovered in urine and faeces.

The phrase "systemic presence of the active ingredient" or "systemic administration" are interchangeable phrases and are meant to include any form of administration of the active ingredient resulting in the entrance of the active ingredient into the blood stream, for example administration by the per-oral, transdermal, transmucosal or the parenteral route.

In a currently interesting embodiment of the invention, the active ingredient is intended for local treatment of the skin and is meant to be administered topically to the skin. Alternatively, the active ingredient is administered to buccal mucosa where it is intended for treating oral pathologies. The treatment is preferentially accomplished by topical application of the active ingredient as defined herein to the affected skin areas or buccal mucous areas for local treatment of the skin and the mucosa, respectively. In such embodiments, the systemic absorption following the topical application should be limited or nil.

In the case of systemic administration the daily total dose would typically be in the range of 0.000001-5 mg/(kg body weight) depending on the duration of the treatment, the pharmaceutical formulation and the bioavailability following per-oral administration, transdermal, transmucosal or parenteral administration. The skilled person will appreciate that the total daily dose may be divided into one or more doses, such as two doses per day or three doses per day.

In the case of topical administration the daily dose of the active ingredient is defined according to the concentration of the active ingredient in the topically administrable composition. The concentration of the active ingredient is typically in the range of 0.0001 - 50.0 % (w/w) depending on the duration of the treatment, the type of formulation and the number of times that the topical composition is to be applied daily. Typically, the concentration of the active ingredient in the topical administrable composition ranges between 0.01% and 25% by weight, preferably 0.02% and 15% by weight, more preferably 0.05% and 12% by weight, such as about 0.05%, 0.1%, 0.2%, 0.25%, 0.4%, 0.5%, 0.6%, 0.75%, 0.8%, 1%, 1.5%, 2%, 2.5%, 3%, 3.5%, 4%, 4.5%, 5%, 5.5%, 6%, 7%, 8%, 9%, 10%, 11%, and 12% by weight of the composition.

The active ingredient may be formulated into several various dosage forms, such as a cream, a lotion, an emulsion, a gel, a solution, a liniment, an ointment, a pasta, a spray, an aerosol, a foam, a liquid or a powder.

### Topically administrable compositions

A second aspect of the invention relates to a dermatological /topically administrable pharmaceutical composition for use in the treatment of the diseases mentioned herein, such as acne. The composition comprises as therapeutically active ingredient
i) Pemirolast or a closely related compound thereof or a pharmaceutically acceptable salt thereof in an amount of at least 0.1% by weight; and further comprises
ii) one or more dermatologically acceptable excipients or carriers.

The concentration of the therapeutically active ingredient is typically in the range of 0.0001 - 50.0 % (w/w) depending on the duration of the treatment, the type of formulation and the number of times that the topical composition is to be applied daily. Typically, in order to achieve satisfactorily affect the concentration of the active ingredient in the topical administrable composition is at least 0.02%, 0.025%, 0,03%, 0.05%, 0.1%, 0.2%, 0.25%, 0.4%, 0.5%, 0.6%, 0.75%, 0.8%, 1%, 1.5%, 2%, 2.5%, 3%, 3.5%, 4%, 4.5%, 5%, 5.5%, 6%, 7%, 8%, 9%, 10%, 11%, and 12% by weight of the composition, but less than 20% by weight due to safety and cost considerations. Thus, in various embodiments, the concentration of the active ingredient ranges between 0.05% and 20% by weight, such as between 0.1% and 20%, such as between 0.2% and 20%, such as between 0.3% and 20%, such as between 0.5 and 20%, such as between 1% and 20% by weight. In still further embodiments thereof, the upper limit concentration of 20% is replaced with 15 or 10% by weight of the composition.

The pharmaceutically acceptable salt and the closely related compound may be selected from any of the salts mentioned under the section describing the therapeutically active ingredient. In one embodiment, the salt is provided as the zinc salt of Pemirolast or a closely related compound thereof.

The skilled person will appreciate that topically administrable compositions of the invention may be in any form suitable for being topically applied to skin or mucosa and with the intention to avoid or at least minimise systemic absorption of the Pemirolast or a closely related compound thereof or a pharmaceutically acceptable salt thereof.

Accordingly, the composition may be in the form of an emulsion, such as a cream, lotion, emulsion, a gel, a solution, a liniment, an ointment, pasta, a spray, an aerosol, a foam, a liquid or a powder. Such compositions are preferably formulated in a manner that limits the systemic uptake, e.g. such that less than 15% by weight, such as less than 10%, 8%, 5% and 3% by weight, of the topically administered Pemirolast or a closely related compound of the invention enters the blood stream following topical administration to the skin or is recovered in urine and faeces.

In various embodiments of the invention, the dermatological composition is intended to be administered directly to skin, such as for the local treatment of skin areas affected by a disease mentioned herein. Therefore, any composition, such as solutions and ointments intended to be administered to the eye are not considered the scope of this invention.

In other embodiments of the invention, a systemic uptake is tolerable. Therefore, in some embodiments, topically administrable formulations for transdermal administration of the active ingredient may be within the scope of the invention.

The pharmaceutical compositions of the invention may be formulated in any solid, semi-solid or fluid form suitable for being administered topically according to conventional pharmaceutical practice, see, e.g., "Remington: The science and practice of pharmacy" 20th ed. Mack Publishing, Easton PA, 2000 ISBN 0-912734-04-3 and "Encyclopaedia of Pharmaceutical Technology", edited by Swarbrick, J. & J. C. Boylan, Marcel Dekker, Inc., New York, 1988 ISBN 0-8247-2800-9.

In one embodiment, the topical administration according to the invention is especially suitable for treating locally affected areas of the mammal, such as the gingiva in the case of periodontitis, peri-implantitis, root caries or root canal treatment, the skin in the case of acne or psoriasis or skin diseases involving tissue destruction such as bed sores, varicose ulcers, etc.

Pemirolast or a closely related compound thereof or a pharmaceutically acceptable salt thereof may be formulated as a cream. Creams are oil-in-water emulsions that contain more than 50% water. As oily base material there are used especially fatty alcohols, for example lauryl, cetyl or stearyl alcohol, fatty acids, for example palmitic or stearic acid, liquid to solid waxes, for example isopropyl myristate, wool wax or beeswax, and/or hydrocarbons, for example vaseline (petrolatum) or paraffin oil. Suitable emulsifiers are surface-active substances having predominantly hydrophilic properties, such as corresponding non-ionic emulsifiers, for example fatty acid esters of polyalcohols or ethylene oxide adducts thereof, such as polyglycerol fatty acid esters or polyoxyethylene sorbitan fatty acid esters (Tweens), also polyoxyethylene fatty alcohol ethers or fatty acid esters, or corresponding ionic emulsifiers, such as alkali metal salts of fatty alcohol sulphates, for example sodium lauryl sulphate, sodium cetyl sulphate or sodium stearyl sulphate, which are customarily used in the presence of fatty alcohols, for example cetyl alcohol or stearyl alcohol. Additives to the aqueous phase are, inter alia, agents that reduce drying out of the creams, for example polyalcohols, such as glycerol, sorbitol, propylene glycol and/or polyethylene glycols, and also preservatives, perfumes, etc.

Ointments are water-in-oil emulsions that contain up to 70%, but preferably from approximately 20% to approximately 50%, water or aqueous phases. Suitable as fatty phase are especially hydrocarbons, for example vaseline, paraffin oil and/or hard paraffins, which preferably contain suitable hydroxy compounds, such as fatty alcohols or esters thereof, for example cetyl alcohol or wool wax alcohol or wool wax, in order to improve their capacity to bind water. Emulsifiers are corresponding lipophilic substances, such as sorbitan fatty acid esters (Spans), for example sorbitan oleate and/or sorbitan isostearate. Additives to the aqueous phase are, inter alia, moisture-retaining agents, such as polyalcohols, for example glycerol, propylene glycol, sorbitol and/or polyethylene glycol, and preservatives, perfumes, etc..

Fatty ointments are anhydrous and contain as base material especially hydrocarbons, for example paraffin, vaseline and/or liquid paraffins, and natural or partially synthetic fats, for example coconut fatty acid triglyceride, or preferably hardened oils, for example hydrogenated groundnut or castor oil, and fatty acid partial esters of glycerol, for example glycerol mono- or di-stearate, and also, for example, the fatty alcohols that increase the water absorption capacity and the emulsifiers and/or additives mentioned in connection with the ointments.

Pastes are creams and ointments with secretion-absorbing powder constituents, such as metal oxides, for example titanium oxide or zinc oxide, also talc and/or aluminium silicates, the function of which is to bind any moisture or secretions present.

In the case of gels, a distinction is made between aqueous and anhydrous or low-water-content gels which consist of swellable, gel-forming materials. There are used especially transparent hydrogels based on inorganic or organic macromolecules. High molecular weight inorganic components having gel-forming properties are predominantly water-containing silicates, such as aluminium silicates, for example betonite, magnesium aluminium silicate, for example veegum, or colloidal silica, for example aerosil. As high molecular weight organic substances there are used, for example, natural, semi-synthetic or synthetic macromolecules. Natural and semi-synthetic polymers are derived, for example, from polysaccharides having very varied carbohydrate building blocks, such as celluloses, starches, tragacanth, gum arabic, agar-agar, gelatine, alginic acid and salts thereof, for example sodium alginate, and derivatives thereof, such as lower alkylcelluloses, for example methyl- or ethyl-celluloses, and carboxy-or hydroxy lower alkylcelluloses, for example carboxymethyl- or hydroxyethyl-celluloses. The building blocks of synthetic, gel-forming macromolecules are, for example, correspondingly substituted unsaturated aliphatic compounds, such as vinyl alcohol, vinylpyrrolidine, acrylic acid or methacrylic acid. As examples of such polymers there may be mentioned polyvinyl alcohol derivatives, such as polyviol, polyvinylpyrrolidines, such as collidine, polyacrylates and polymethacrylates, such as Rohagit S or Eudispert. Customary additives, such as preservatives or perfumes, may be added to the gels.

Foams are administered, for example, from pressurised containers and are oil-in-water emulsions in aerosol form, there being used as propellants halogenated hydrocarbons, such as chlorofluoro-lower alkanes, for example dichlorodifluoromethane or dichlorotetrafluoroethane. As oily phase there are used, inter alia, hydrocarbons, for example paraffin oil, fatty alcohols, for example cetyl alcohol, fatty acid esters, for example isopropylmyristate, and/or other waxes. As emulsifiers there are used, inter alia, mixtures of those having predominantly hydrophilic properties, such as polyoxyethylene sorbitan fatty acid esters (Tweens), and those having predominantly lipophilic properties, such as sorbitan fatty acid esters (Spans). The customary additives, such as preservatives, etc. are added thereto.

Tinctures and solutions have in most cases an aqueous-ethanolic base to which have been added, inter alia, polyalcohols, for example glycerol, glycols and/or polyethylene glycol, as moisture-retaining agents to reduce evaporation and fat-restoring substances, such as fatty acid esters with low molecular weight polyethylene glycols, that is to say lipophilic substances that are soluble in aqueous mixture as a replacement for the fatty substances removed from the skin with the ethanol, and, if necessary, other adjuncts and additives.

Specific dental preparations include mouth rinse or mouth wash liquids, tooth pastes, locally cementing gels, dental floss, guided tissue regeneration (GTR) membranes, chewing gums, periodontal or surgical pastes, laquers, etc. Especially in the case of periodontal diseases it may be preferable to incorporate the Pemirolast or a closely related compound thereof or a pharmaceutically acceptable salt thereof into a polymeric carrier which may be delivered therein directly to the afflicted area. The periodontal pockets may also be topically treated with Pemirolast or a closely related compound thereof or a pharmaceutically acceptable salt thereof preparations by various techniques either at home by home care devices or at the dentist by chair-side devices such as ultrasonic systems.
Aerosol or spray preparations may be used for application to wounds or cancer and for preventing metastases.

The dermatologically administrable pharmaceutical preparations are prepared in a manner known per se by mixing with pharmaceutical adjuncts that are customary for that purpose, for example by dissolving or suspending the active ingredient in the base material or in a portion thereof, if necessary In order to prepare emulsions in which the active ingredient is dissolved in one of the liquid phases, the active ingredient is, as a rule, dissolved therein before the emulsification; in order to prepare suspensions in which the active ingredient is suspended in the emulsion, the active ingredient is mixed with a portion of the base material after the emulsification and then added to the remainder of the formulation.

In dermatological compositions of the invention, the Pemirolast or a compound thereof will usually be distributed in a liquid carrier system such as water or any aqueous solution containing organic or inorganic materials. Additionally, the compositions may contain one or more ingredients to modify or enhance their texture, appearance, scent performance or stability. Illustrative additives to the compositions include: ointment bases, solvents, buffering agents, pH-adjusting agents, preservatives, humectants, chelating agents, antioxidants, stabilizers, emulsifying agents, suspending agents, gel-forming agents, perfumes, skin protective agents, Lubricants, such as silicones or isopropyl myristate, astringents such as lactic acid or zinc phenolsulfonate, fragrances such as bay oil, rose water, orange oil, myrrh or musk, antiseptics such as menthol and camphor, emollients such as glycerols and sorbitols, and preservatives such as ascorbic acid, parahydroxybenzoic acid esters and tocopherols.

Typical ointment bases may be selected from the group comprising white vaseline, beeswax, paraffin, cetanol, cetyl palmitate, vegetable oils, sorbitan esters of fatty acids (Span), polyethylene glycols, and condensation products between sorbitan esters of fatty acids and ethylene oxide, e.g. polyoxyethylene sorbitan monooleate (Tween, such as Tween 80).

Typical hydrophobic ointment bases may be selected from the group comprising paraffin's, vegetable oils, animal fats, synthetic glycerides, waxes, lanolin, and liquid polyalkylsiloxanes. Typical hydrophilic ointment bases are but not limited to solid macrogols (polyethylene glycols).

Typical solvents may be selected from the group comprising water; alcohols such as, polyethylene glycols; propylene glycols; glycerol; liquid polyalkylsiloxanes; and mixtures thereof.

Typical buffering agents may be selected from the group comprising of citric acid, acetic acid, tartaric acid, lactic acid, hydrogen phosphoric acid, diethylamine etc. Typical pH-adjusting agents are organic and inorganic acids and bases, such as potassium or sodium hydroxide monoethanolamine, triethanolamine and hydrochloric acid.

Typical preservatives may be selected from the group comprising parabens, such as methyl, ethyl, propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalconium chloride, and benzyl alcohol, or mixtures of preservatives.

Typical humectants may be selected from the group comprising glycerin, propylene glycol, sorbitol, lactic acid, urea, and mixtures thereof. Typical chelating agents are but not limited to sodium EDTA and citric acid. Typical antioxidants may be selected from the group comprising butylated hydroxy anisole (BHA), ascorbic acid and derivatives thereof, tocopherol and derivatives thereof, cysteine, and mixtures thereof. Suitable emulsifying agents may be selected from the group comprising naturally occurring gums, e.g. gum acacia or gum tragacanth; naturally occurring phosphatides, e.g. soybean lecithin; sorbitan monooleate derivatives; wool fats; wool alcohols; sorbitan esters; monoglycerides; fatty alcohols, fatty acid esters (e.g. triglycerides of fatty acids); and mixtures thereof.

Suitable suspending agents may be selected from the group comprising celluloses and cellulose derivatives such as, e.g., carboxymethyl cellulose, hydroxyethylcellulose, hydroxypropylcellulose, hydroxypropylmethylcellulose, carrageenan, acacia gum, arabic gum, tragacanth, and mixtures thereof.

Suitable gel bases and viscosity-increasing components may be selected from the group comprising liquid paraffin, polyethylene, fatty oils, colloidal silica or aluminium, zinc soaps, glycerol, propylene glycol, tragacanth, carboxyvinyl polymers, magnesium-aluminium silicates, Carbopol®, hydrophilic polymers such as, e.g. starch or cellulose derivatives such as, e.g., carboxymethylcellulose, hydroxyethylcellulose and other cellulose derivatives, water-swellable hydrocolloids, carrageenans, hyaluronates (e.g. hyaluronate gel optionally containing sodium chloride), and alginates including propylene glycol alginate.

### Further therapeutically active ingredients

Further ingredients, either therapeutically active ingredients or dermatologically acceptable ingredients can be co-administered together with Pemirolast or a closely related compound thereof or a salt thereof, or added to a medicament of the invention, such as to a topically administrable composition of the invention (dermatological composition) in order to strengthen, improve, potentiate, or prolong the therapeutic action demonstrated herein or to provide a less toxic, safer, more convenient, better tolerated, or less expensive treatment approach. For example, in the treatment of acne it is usual to apply combination therapy.

Therefore, in some embodiments of the invention, medicaments, methods, uses and dermatological compositions further comprises one or more additional therapeutically active agent(s). For example, the additional therapeutically active agent may be selected from those generally applied in the treatment of acne (anti-acne agents) or in the treatment of psoriasis, chronic ulcers, scars, burns, skin cancer, skin ageing, rosacea, seborrhea and seborrheic dermatitis.

In considering the treatment of acne, uses and methods of the invention further comprising administering in combination or by co-administering a treatment agent usually applied in the treatment of acne, such as salicylic acid, nicotinamide, azelaic acid, a zinc compound, an antibiotic, a retinoid, an oral contraceptive and an anti-androgen.

Accordingly, a topically administrable composition of the invention may further comprise a treatment agent for acne selected from the group consisting of salicylic acid, nicotinamide, azelaic acid, a zinc compound, an antibiotic, a retinoid, an oral contraceptive and an anti-androgen.

Typical examples for use as an antibiotic are those antibiotics that reduce the population of P. acnes in skin. To be mentioned is azelaic acid, benzoyl peroxide, erythromycin, clindamycin, tretracycline, doxycycline and minocycline, trimethoprim, sulfamethoxazole, and azithromycine.

Typical examples for use as a retinoid are adapalene, tazarotene, tretinoin, and isotretinoin.

A typical example on an anti-androgen is spironolactone.

A zinc compound may typically be selected from the group consisting of water soluble, poorly water soluble and water insoluble zinc salts, compounds and complexes, such as zinc acetate, zinc bacitracin, zinc bromide, zinc cysteate, zinc caprylate, zinc chloride, zinc citrate, zinc fluoride, zinc formate, zinc glycinate, zinc iodate, zinc lactate, zinc nitrate, zinc nitrite, zinc oleate, zinc oxalate, zinc oxide, zinc permanganate, zinc peroxide, zinc phenolsulfonate, zinc phosphate, zinc propionate, zinc pyrophosphate, zinc ricinoleate, zinc salicylate, zinc selenate, zinc silicate, zinc selenide, zinc sulfate, zinc stearate, zinc sulfide, zinc tannate, zinc tartrate, zinc valerate, zinc peptides, and zinc protein complexes. In a preferred embodiment of the present invention, the zinc compound is zinc lactate or zinc acetate.

Furthermore, in some embodiments of the invention, medicaments and dermatological compositions of the invention further comprises minerals, skin nutrients, amino acids, ethanolamine, glucosamine.

### EXAMPLES

### Example 1. Topically administrable composition of Pemirolast

Solutions and gel formulations comprising 0.5%, 1%, 2% or 4% by weight of Pemirolast (as the potassium salt) were prepared according to methods well-known in the art.

### Example 2. Inhibition of disease mediators

The inhibitory effect of Pemirolast on human MMP's and cytokines were determined by enzyme assays carried out by MDS Pharma Services.

The inhibition of MMP's can be determined according to the test methods described by Knight CG, Willenbrock F, Murphy G (A novel coumarin-labelled peptide for sensitive continuous assays of the matrix metalloproteinases, FEBS Lett. 1992 Jan 27;296(3):263-6); Johnson LL, Dyer R, Hupe DJ (Matrix metalloproteinases, Curr Opin Chem Biol. 1998 Aug;2(4):466-71); and Olson MW, Gervasi DC, Mobashery S, Fridman R (Kinetic analysis of the binding of human matrix metalloproteinase-2 and -9 to tissue inhibitor of metalloproteinase (TIMP)-1 and TIMP-2, J Biol Chem. 1997 Nov 21;272(47):29975-83). The methods employ Human rheumatoid synovial fibroblast (MMP-1) and Human recombinant (MMP-2 and MMP-3) and Human recombinant (E.coli) (MMP-7) as the MMP source. The substrate is Mca-Pro-Leu-Gly-Leu-Dpa-Ala-Arg-NH2. The enzymatic reaction is based on the conversion of Mca-Pro-Leu-Gly-Leu-Dpa-Ala-Arg to Mca-Pro-Leu-Gly + Leu-Dpa-Ala-Arg-NH2, where Mca-Pro-Leu-Gly is quantified using spectrofluorimetric quantitation. As reference compounds are employed TIMP-1 and TIMP-2. Such methods are commercially available at MDS Pharma Services.

The inhibitory effect of Pemirolast on PPAR was determined by Indigo Biosciences. The following assay procedure was used: Compounds were diluted in DMSO to a final concentration of 100 mM and serial dilutions prepared. The cell line HEK 293-T was transiently transfected with Gal4-human PPARα, γ, Gal4-luciferase and pRL (renilla, to correct for efficiency). The cells were treated with Pemirolast or with positive controls (ciprofibrate, or rosiglitazone) for 14 hours. The cells were lysed and luciferase and renilla activity were determined.

The inhibition of Pemirolast on cytokine production was determined using the assay "LPS induced human PBMC".

| Enzyme | Concentration of Pemirolast | % inhibition |
|---|---|---|
| MMP-1 | 18 µM | 50 |
| MMP-2 | 25 µM | 50 |
| MMP-3 | 32 µM | 50 |
| MMP-7 | 32 µM | 50 |
| PPAR-α | 17 µM | 50 |
| PPAR-γ | 20 µM | 50 |
| TNF-α | 100 µM | 50 |

### Example 3. Anti-inflammatory activity

The anti-inflammatory potential of Pemirolast can be tested in "arachidonic acid-induced ear-inflammation test in mice", a commonly employed method for screening and evaluation of antiinflammatory drugs. Dexamethasone was employed as reference compound.

The study can be performed in female BALB/cA mice from M & B A/S, DK-8680 Ry. The test substances (various concentration of Pemirolast dissolved in aqueous solutions) and the reference compound are administered intraperitoneally in volumes of 20 ml per kg body weight 30 minutes before application of arachidonic acid to the ear. All groups are then treated with 20 µl arachidonic acid, 100 mg/ml in acetone, on the right ear.
One hour after the application of arachidonic acid the mice are sacrificed, the ears cut from the tip with a punch biopsy knife (8 mm diameter) and weighed. Mean weights and standard deviations are calculated. Relative ear oedema is assessed as the weight difference between right and left ear of each mouse expressed as percent of the left ear. Percent inhibition of the relative ear oedema compared with the vehicle treated groups is calculated for the test substance and reference compound treated groups. Differences in relative ear oedema between the vehicle treated groups and the test substance and reference compound treated groups are tested for significance employing a non-parametric statistical method of analysis, the Mann-Whitney U test. The required level of significance is p<0.05.

Arachidonic acid causes an inflammation in the ears, which inflammation is visible after about 30 minutes. It was demonstrated that Pemirolast significantly prevented the inflammatory reaction in the arachidionic acid treated ear.

### Example 4. Case with treatment of acne vulgaris

A 39 years old woman was suffering from acne vulgaris characterised by comedones and some inflammatory lesions. She had previously regularly used a 5% benzoyl peroxide cream to treat the condition.
During a flare up of the condition she applied a 5.0% aqueous solution of Pemirolast (potassium salt) once daily for four weeks to the areas affected. After four weeks of treatment the inflammatory lesions were completely recessed and the inflamed area surrounding the lesion was also recessed without any noticeable scarring. Notably, the usual treatment with benzoyl peroxide did not remove the reddened inflamed area. The treatment was followed and assessed by a physician.

## Claims

1. Use of Pemirolast or a closely related compound thereof or a pharmaceutically salt thereof for the preparation of a medicament for the treatment of a disease **characterised by** the presence of inflammation together with activity of matrix metalloproteinases and/or peroxisome proliferator-activated receptors selected from the group consisting of acne, acne scarring, psoriasis, chronic ulcers, scars, burns, skin cancer, skin ageing, rosacea, seborrhea and seborrheic dermatitis, wherein the closely related compound thereof is defined by formula II, 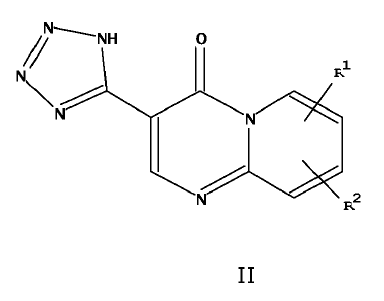 wherein R¹ and R² each independently designate radicals selected from the group consisting of hydrido (H), optionally substituted C₁₋₆-alkyl, C₄₋₆-cycloalkyl, C₂₋₆-alkenyl, C₂₋₆-alkynyl, C₁₋₆-alkoxyl, phenyl, C₇₋₁₄alkaryl, C₇₋₁₄alkheterocyclyl, carboxyl (COOH), carboxyl derivative (COOR'), cyano (CN), CF₃, halogen (Br, Cl, FI, I), hydroxy (OH), hydroxy derivative (OR'), primary amino (NH₂), secondary amino (NHR'), tertiary amino (NR'R"), carboxy (CO), carboxy derivative (CO- R'), and wherein R' and R" independently defines a radical selected from C₁₋₆-alkyl, C₄₋₆-cycloalkyl, C₂₋₆-alkenyl, C₂₋₆-alkynyl, C₁₋₆-alkoxyl, phenyl, C₇₋₁₄alkaryl and C₇₋₁₄alkheterocyclyl; and wherein R¹ and R² may be located at any of positions 6, 7, 8 or 9 of the pyrido[1,2-a]pyrimidine ring system.

2. The use according to claim 1, wherein the disease is acne or any clinical variant thereof or acne scarring.

3. The use according to claim 1, wherein the disease is psoriasis or any clinical variant thereof.

4. The use according to claim 1, wherein the disease is seborrhea.

5. The use according to claim 1, wherein the disease is seborrheic dermatitis.

6. The use according to claim 2, wherein the medicament further comprises a treatment agent selected from the group consisting of salicylic acid, nicotinamide, azelaic acid, a zinc compound, an antibiotic, a retinoid, an oral contraceptive and an anti-androgen.

7. The use according to any one of the preceding claims, wherein the medicament is formulated for topical administration to skin or for buccal administration to the mucosa.

8. The use according to claim 7, wherein the medicament is formulated as a gel, solution, emulsion, foam, liniment or ointment.

9. A topically administrable pharmaceutical composition comprising as therapeutically active ingredient Pemirolast or a closely related compound thereof or a pharmaceutically acceptable salt thereof in an amount of at least 0.1% by weight; and further comprising one or more dermatologically acceptable excipients or carriers.

10. The composition according to claim 9, further comprising a treatment agent selected from the group consisting of salicylic acid, nicotinamide, azelaic acid, a zinc compound, an antibiotic, a retinoid, an oral contraceptive and an anti-androgen.
